# EUROPEAN PATENT APPLICATION

(11) **EP 4 501 331 A1**
(43) Date of publication of application: **05.02.2025**
(21) Application number: 23780694.8
(22) Date of filing: 29.03.2023
(51) Int. Cl.: A61K 31/663, A61P 19/00, A61P 19/02, A61P 19/08

(54) **AGENT FOR INDUCING PROLIFERATION OR DIFFERENTIATION OF CHONDROCYTE**

(30) Priority: 31.03.2022 JP 2022061383
(71) Applicant: Tohoku University, Sendai-shi, Miyagi 980-8577 (JP)
(72) Inventor: MIKI CHIBA, Mirei, Sendai-shi, Miyagi 980-8577 (JP); TAKAHASHI, Tetsu, Sendai-shi, Miyagi 980-8577 (JP); MIZOGUCHI, Itaru, Sendai-shi, Miyagi 980-8577 (JP); SHINODA, Hisashi, Sendai-shi, Miyagi 980-8577 (JP)
(74) Representative: Winter, Brandl - Partnerschaft mbB
(86) International application number: PCT/JP2023/012799
(87) International publication number: WO 2023/190685

(57) **Abstract**

A problem to be solved by the invention is to provide a novel agent for inducing proliferation or differentiation of a chondrocyte containing a compound which has not been known so far to have the capability of inducing proliferation or differentiation of a chondrocyte as an active ingredient. The invention provides an agent for inducing proliferation or differentiation of a chondrocyte containing the compound (1) or a salt thereof.

## Description

### Technical Field

### [Cross-Reference to Related Application]

The present application claims priority to Japanese patent application No. 2022-061383 description filed on March 31, 2022 (the entire disclosure thereof is incorporated in the present description by reference). The present invention relates to an agent for inducing proliferation or differentiation of a chondrocyte and a method for inducing proliferation or differentiation of a chondrocyte.

### Background Art

Temporomandibular disorder is a disorder caused by a problem in the muscles or the joints of the jaws or in the fibrous tissue connecting these and causes symptoms such as headaches, oppressive pain in the muscles of mastication and a clicking sound from the jaw joints. The estimated number of patients with any symptom in the jaw joints is said to be as high as about 19 million, and development of a therapeutic method thereof is desired.

In temporomandibular disorder, the cartilage in a joint is sometimes shifted or damaged, and regeneration of the cartilage, if possible, is effective for treating temporomandibular disorder. Moreover, cartilage is a connective tissue composed of chondrocytes and a surrounding matrix and is found abundantly in the parts where bones are connected such as joints. There are many disorders caused by damage or the like of the cartilage also in these joints and the like, and regeneration of cartilage, if possible, is expected to be able to be a useful option for treating these disorders.

### Citation List

### Patent Literature

PTL 1: JP5655179B

### Non Patent Literature

NPL 1: Mayo Clin Proc. 83, (2008) p1032-1045
NPL 2: Journal of Pharmacological Sciences 131 (2016) p37-50.
NPL 3: nature review immunology, 2008, p.727

### Summary of Invention

### Technical Problem

The invention provides a novel agent for inducing proliferation or differentiation of a chondrocyte containing a compound which has not been known so far to have the capability of inducing proliferation or differentiation of a chondrocyte as an active ingredient.

### Solution to Problem

Under the circumstances, as a result of examination of a wide variety of compounds, the present inventors have found that a compound represented by the general formula (1) or a salt thereof, which is described below, unexpectedly has the capability of inducing proliferation or differentiation of a chondrocyte. The invention is based on the new findings.

Therefore, the invention provides the items below.

Item 1. An agent for inducing proliferation or differentiation of a chondrocyte containing a compound represented by the general formula (1) or a salt thereof 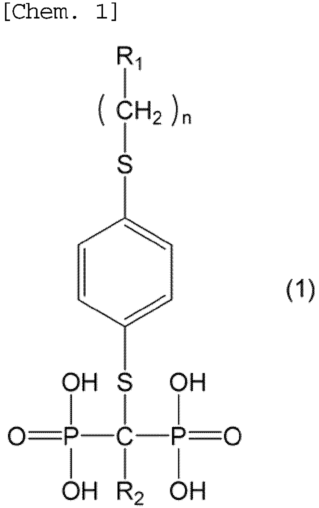 [wherein in the formula, R₁ represents a hydrocarbon group of C1 to C5 which may be substituted, R₂ represents hydrogen or a hydroxy group, and n represents an integer of 0 to 3].

Item 2. A prophylactic or therapeutic agent for a disorder which can be treated by induction of proliferation or differentiation of a chondrocyte containing a compound represented by the general formula (1) or a salt thereof 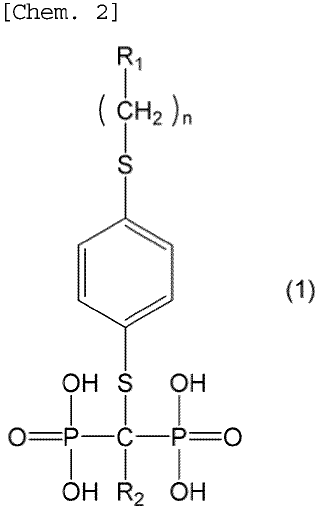 [wherein in the formula, R₁ represents a hydrocarbon group of C1 to C5 which may be substituted, R₂ represents hydrogen or a hydroxy group, and n represents an integer of 0 to 3].

Item 3. The prophylactic or therapeutic agent according to item 2, wherein the disorder which can be treated by induction of proliferation or differentiation of a chondrocyte is osteoarthritis, inflammatory arthritis, cartilage damage, congenital cartilage defect, an age-related decrease in articular cartilage or chondrodystrophy.

Item 4. The prophylactic or therapeutic agent according to item 2 or 3 which is systemically administered or locally administered.

Item 5. The agent for inducing proliferation or differentiation of a chondrocyte according to item 1 or the prophylactic or therapeutic agent according to any one of items 2 to 4, wherein the compound represented by the general formula (1) or the salt thereof is [4-(methylthio)phenylthio]-methanebisphosphonic acid or a salt thereof.

Item 6. A method for inducing proliferation or differentiation of a chondrocyte including a step of causing a compound represented by the general formula (1) or a salt thereof to act on a mesenchymal stem cell and/or a cartilage precursor cell *in vitro* 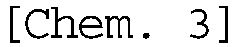 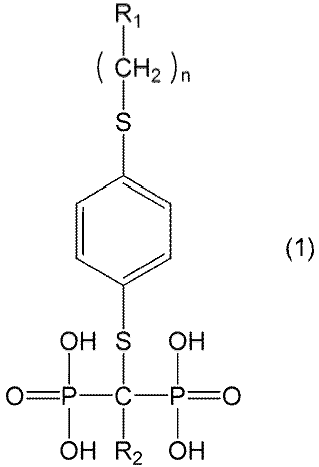 [wherein in the formula, R₁ represents a hydrocarbon group of C1 to C5 which may be substituted, R₂ represents hydrogen or a hydroxy group, and n represents an integer of 0 to 3].

Item 7. Use of a compound represented by the general formula (1) or a salt thereof for the manufacture of a prophylactic or therapeutic agent for a disorder which can be treated by induction of proliferation or differentiation of a chondrocyte: 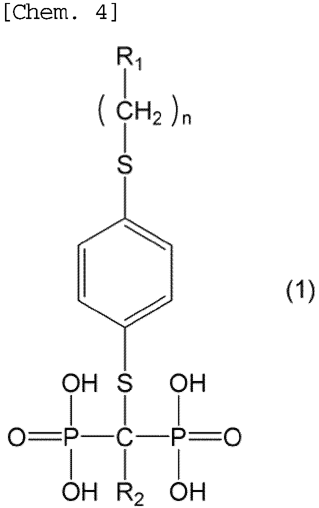 [wherein in the formula, R₁ represents a hydrocarbon group of C1 to C5 which may be substituted, R₂ represents hydrogen or a hydroxy group, and n represents an integer of 0 to 3].

Item 8. The use according to item 7, wherein the disorder which can be treated by induction of proliferation or differentiation of a chondrocyte is osteoarthritis, inflammatory arthritis, cartilage damage, congenital cartilage defect, an age-related decrease in articular cartilage or chondrodystrophy.

Item 9. The use according to item 7 or 8 which is systemically administered or locally administered.

Item 10. The agent for inducing proliferation or differentiation of a chondrocyte according to item 1 or the use according to any one of items 7 to 9, wherein the compound represented by the general formula (1) or the salt thereof is [4-(methylthio)phenylthio]-methanebisphosphonic acid or a salt thereof.

Item 11. A compound represented by the general formula (1) or a salt thereof for use in the prevention or the treatment of a disorder which can be treated by induction of proliferation or differentiation of a chondrocyte: 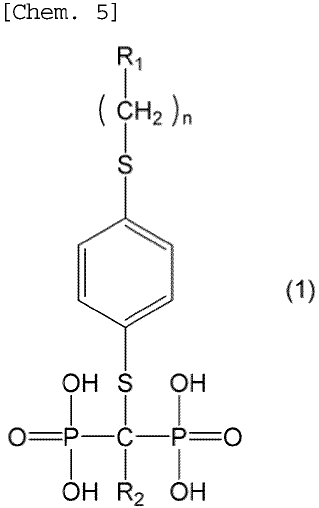 [wherein in the formula, R₁ represents a hydrocarbon group of C1 to C5 which may be substituted, R₂ represents hydrogen or a hydroxy group, and n represents an integer of 0 to 3].

Item 12. The compound or the salt thereof for use according to item 11, wherein the disorder which can be treated by induction of proliferation or differentiation of a chondrocyte is osteoarthritis, inflammatory arthritis, cartilage damage, congenital cartilage defect, an age-related decrease in articular cartilage or chondrodystrophy.

Item 13. The compound or the salt thereof for use according to item 11 or 12 which is systemically administered or locally administered.

Item 14. The agent for inducing proliferation or differentiation of a chondrocyte according to item 1 or the compound or the salt thereof for use according to any one of items 11 to 13, wherein the compound represented by the general formula (1) or the salt thereof is [4-(methylthio)phenylthio]-methanebisphosphonic acid or a salt thereof.

### Advantageous Effects of Invention

According to the invention, using a compound represented by the general formula (1), which is a compound that has not been known so far to have the effect of proliferating or differentiating a chondrocyte, or a salt thereof as an active ingredient, a novel agent for inducing proliferation or differentiation of a chondrocyte can be provided. Moreover, according to the invention, using the compound represented by the general formula (1) or a salt thereof as an active ingredient, a novel prophylactic or therapeutic agent for a disorder which can be treated by induction of proliferation or differentiation of a chondrocyte can be provided.

### Brief Description of Drawings

[Fig. 1] Pictures of the hematoxylin-eosin-stained tissues of the mandibular condylar cartilage in Example 1 are shown. The thickness of the cartilage layer (the chondrocyte layer + the hypertrophic chondrocyte layer) was increased by the administration of MPMBP. F: fibrous layer, P: proliferating cell layer, C: chondrocyte layer, and H: hypertrophic chondrocyte layer.
[Fig. 2] The histomorphometric results of the effects of the OVX and the subsequent administration of MPMBP on the mandibular condylar cartilage in Example 1 are shown. The thickness of the cartilage layer (the chondrocyte layer + the hypertrophic chondrocyte layer) was increased by the administration of MPMBP. F: fibrous layer, P: proliferating cell layer, C: chondrocyte layer, and H: hypertrophic chondrocyte layer.
[Fig. 3] The tissue images of the toluidine blue-stained mandibular condylar cartilage in Example 1 are shown. In the toluidine blue staining-positive cartilage layer (the hypertrophic chondrocyte layer) which was reduced by the OVX, the intensity of the toluidine blue staining was increased in a dose-dependent manner by the administration of MPMBP, and the thickness of the stained cartilage layer increased. F: fibrous layer, P: proliferating cell layer, C: chondrocyte layer, and H: hypertrophic chondrocyte layer.
[Fig. 4] The results of evaluation of the effects of the OVX and the subsequent administration of MPMBP in the mandibular condylar cartilage matrix using toluidine blue-stained slices in Example 1 are shown. The thickness of the toluidine blue staining-positive cartilage layer (the hypertrophic chondrocyte layer) which was reduced by the OVX was increased by the administration of MPMBP. F: fibrous layer, P: proliferating cell layer, C: chondrocyte layer, and H: hypertrophic chondrocyte layer.
[Fig. 5] The results of immunostaining in Example 1 are shown. The chondrocyte layer of the mandibular condylar chondrocytes of the OVX rats was proliferated by the administration of MPMBP.
[Fig. 6] The results of the proliferation test of the ATDC5 cells to which various compounds were added in Example 2 are shown. MPMBP significantly proliferated the culture chondrocyte ATDC5 cells.
[Fig. 7] The results of the viability test of the ATDC5 cells to which various compounds were added in Example 2 are shown. MPMBP increased the viability of the culture chondrocyte ATDC5 cells.
[Fig. 8] The results of toluidine blue staining of the tibiae in Example 3 are shown. The cartilage layers of the articular cartilage and the growth plate cartilage of the toluidine blue-positive tibiae were increased by the MPMBP treatment.
[Fig. 9] The results of immunohistochemical staining in Example 3 are shown. PCNA-positive cells, which indicate proliferation, were observed in the articular cartilage and the growth plate cartilage of the tibiae of the OVX rats to which MPMBP was administered.

### Description of Embodiments

In the present description, a singular form (a, an, the or the like) includes both a singular form and a plural form unless it is otherwise clearly stated in the present description or unless it is clearly inconsistent in the context.

### Prophylactic or Therapeutic Agent for Disorder

The invention provides a prophylactic or therapeutic agent for a disorder which can be treated by induction of proliferation or differentiation of a chondrocyte containing or a salt thereof. 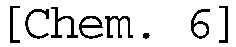 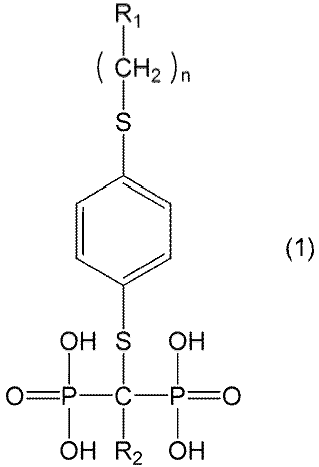 [In the formula, R₁ represents a hydrocarbon group of C1 to C5 which may be substituted. R₂ represents hydrogen or a hydroxy group. n represents an integer of 0 to 3]. In the invention, the compound represented by the general formula (1) is sometimes also simply referred to as the compound (1).

The compound (1), which is the active ingredient of the invention, is known or can be produced from a known compound as a raw material.

The hydrocarbon group in the invention is an alkyl group (for example, methyl group, ethyl group, n-propyl group, i-propyl group, n-butyl group, i-butyl group, t-butyl group, n-pentyl group or the like), an alkenyl group (for example, vinyl group, propenypyl group, butenyl group, pentenyl group or the like), an alkynyl group (for example, ethynyl group, propynypyl group, butynyl group, pentynyl group or the like), a cycloalkyl group (for example, cyclopropyl group, cyclobutyl group, cyclopentyl group or the like) or the like.

The hydrocarbon group represented by R₁ is of C1 to C5, preferably C1 to C3, more preferably C1. When the hydrocarbon group is substituted, the substituent is not limited, but examples thereof include hydroxy group, a halogen, an alkoxy group, and an acetyl group. When the hydrocarbon group represented by R₁ has a substituent(s), the number thereof is not limited but is, for example, one to three, preferably one. The alkoxy group in the invention is an alkoxy group in which the alkyl moiety is a linear or branched alkyl group (for example, methyl group, ethyl group, n-propyl group, i-propyl group, n-butyl group, i-butyl group, t-butyl group, n-pentyl group or the like) or the like, preferably an alkoxy group of C1 to C5, preferably an alkoxy group of C1 to C3, more preferably methoxy group. The halogen in the invention is fluorine, chlorine, bromine, iodine or the like.

The compound (1) or the salt thereof in the invention is preferably tetrahydrogen-4-methylthiophenylthiomethane bisphosphonate or a salt thereof. Tetrahydrogen-4-methylthiophenylthiomethane bisphosphonate is a known substance having the structure below.

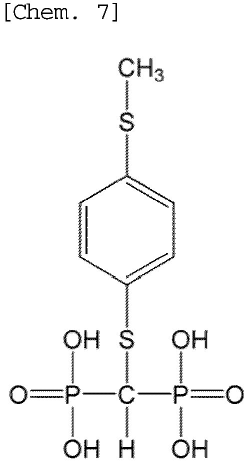

Examples of the salt of the compound (1), which is the active ingredient of the invention, include a salt with a base. Specific examples of the salt with a base include an alkali metal or alkaline earth metal salt such as a sodium salt, a potassium salt and a calcium salt. In the invention, a salt with a base such as an alkali metal salt is preferable, and a sodium salt is further preferable. In the invention, of sodium salts, a disodium salt is preferable, and disodium [4-(methylthio)phenylthio]-methanebisphosphonate (disodium dihydrogen[4(methylthio)pheylthio]methanebisphosphonate, abbreviated to as MPMBP) is particularly preferable.

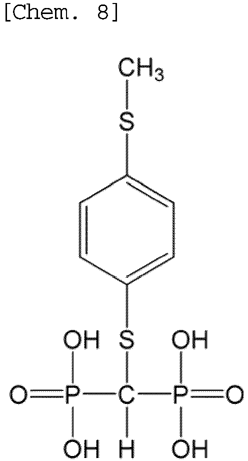

The compound (1) and the salt thereof, which are the active ingredients of the invention, sometimes exist in the form of a hydrate or a solvate, and thus the hydrates and the solvates are also included in the compound as the active ingredient of the invention.

Examples of the solvent which forms a solvate include alcohols such as ethanol and propanol, organic acids such as acetic acid, esters such as ethyl acetate, ethers such as tetrahydrofuran and diethyl ether, ketones such as acetone, and DMSO. One kind of these solvents alone or a mixed solvent of two or more kinds thereof can be used.

Examples of the disorder which can be treated by induction of proliferation or differentiation of a chondrocyte include arthritis such as osteoarthritis and inflammatory arthritis, and congenital cartilage defect or cartilage defect due to injury or inflammation. Examples of the cartilage are the cartilage in the nose, ears, jaws, knees, hip joints, elbows, shoulders, hands, feet, spine and the like. Moreover, the arthritis is, for example, arthritis of the jaws, knees, hip joints, elbows or the like and is typically temporomandibular disorder, knee arthritis or the like. Here, in the present description, the "prophylactic or therapeutic agent for a disorder which can be treated by induction of proliferation or differentiation of a chondrocyte of the invention" is sometimes simply referred to as the "prophylactic or therapeutic agent of the invention".

In the invention, the compound (1) or the salt thereof itself, which is the active ingredient, may be used as the prophylactic or therapeutic agent of the invention or used as a composition combined with various carriers which are acceptable for a pharmaceutical or food application or the like (for example, an isotonizing agent, a chelating agent, a stabilizer, a pH regulator, an antiseptic, an antioxidant, a solubilizer, a thickener or the like).

Examples of the isotonizing agent include saccharides such as glucose, trehalose, lactose, fructose, mannitol, xylitol and sorbitol, polyhydric alcohols such as glycerin, polyethylene glycol and propylene glycol, and inorganic salts such as sodium chloride, potassium chloride and calcium chloride. One kind of these isotonizing agents alone or a combination of two or more kinds thereof can be used.

Examples of the chelating agent include edetates such as disodium edetate, calcium disodium edetate, trisodium edetate, tetrasodium edetate and calcium edetate, ethylenediamine tetraacetate, nitrilotriacetic acid or a salt thereof, sodium hexametaphosphate, and citric acid. One kind of these chelating agents alone or a combination of two or more kinds thereof can be used.

Examples of the stabilizer include sodium hydrogen sulfite.

Examples of the pH regulator include acids such as hydrochloric acid, carbonic acid, acetic acid and citric acid, alkali metal hydroxides such as sodium hydroxide and potassium hydroxide, alkali metal carbonates or hydrogencarbonates such as sodium carbonate, alkali metal acetates such as sodium acetate, alkali metal citrates such as sodium citrate, and bases such as trometamol. One kind of these pH regulators alone or a combination of two or more kinds thereof can be used.

Examples of the antiseptic include sorbic acid, potassium sorbate, paraoxybenzoate esters such as methyl paraoxybenzoate, ethyl paraoxybenzoate, propyl paraoxybenzoate and butyl paraoxybenzoate, quaternary ammonium salts such as chlorhexidine gluconate, benzalkonium chloride, benzethonium chloride and cetylpyridinium chloride, alkylpolyaminoethyl glycine, chlorobutanol, Polyquad, polyhexamethylene biguanide, and chlorhexidine. One kind of these antiseptics alone or a combination of two or more kinds thereof can be used.

Examples of the antioxidant include sodium hydrogen sulfite, dried sodium sulfite, sodium pyrosulfite, and concentrated mixed tocopherols. One kind of these antioxidants alone or a combination of two or more kinds thereof can be used.

Examples of the solubilizer include sodium benzoate, glycerin, D-sorbitol, glucose, propylene glycol, hydroxypropyl methylcellulose, polyvinylpyrrolidone, macrogol, and D-mannitol. One kind of these solubilizers alone or a combination of two or more kinds thereof can be used.

Examples of the thickener include polyethylene glycol, methylcellulose, ethylcellulose, carmellose sodium, xanthan gum, chondroitin sodium sulfate, hydroxyethylcellulose, hydroxypropylcellulose, hydroxypropylmethylcellulose, polyvinylpyrrolidone, and polyvinyl alcohol. One kind of these thickeners alone or a combination of two or more kinds thereof can be used.

Furthermore, in a preferable embodiment, the composition of the invention may be used in combination with a method used for preventing or treating a disorder which can be treated by induction of proliferation or differentiation of a chondrocyte, such as the methods below.

### (1) Antiinflammatory Analgesic and/or Steroid

For example, combination use with diclofenac, naproxen, loxoprofen, etodocla, meloxicam, celecoxib, steroid, aminotriptin, pregabalin or the like.

### (2) Cartilage Regenerative Medicine and/or Cartilage Transplantation Therapy

Combination use with a cartilage regenerative therapy, transplantation or the like such as filling of a defect part with chondrocytes and a cartilage matrix or with a formed carrier containing these.

### (3) Proliferator, Growth Factor, Cytokine and/or Matrix Protein Acting on Chondrocytes

For example, combination use with TGFβ superfamily, BMP, IGF, VEGF, CNP, FGF, a proteoglycan, type II collagen, PTHrP or the like.

### (4) Tissue Damage Suppressor

For example, combination use with an antirheumatic drug or an immunosuppressive agent used for strong inflammation, rheumatism or the like, a biopharmaceutical (fusion protein of an anti-TNFα monoclonal antibody or a soluble TNFα receptor and human IgG), a JAK inhibitor or the like.

### (5) Therapeutic Agent for Osteoporosis

For example, combination use with denosumab, teriparatide, an active vitamin D3 agent, a selective estrogen receptor agonist, romosozumab or the like.

Thus, the composition of the invention may further contain a component used for the combination uses in addition to the compound (1) or the salt thereof. When such a component is used, one kind thereof alone or a combination of two or more kinds thereof can be used.

In the embodiment of the composition, the amount of the compound (1) or the salt thereof contained in the composition is not particularly limited and can be appropriately set from the conditions of, for example, 90 mass% or more, 70 mass% or more, 50 mass% or more, 30 mass% or more, 10 mass% or more, 5 mass% or more, 1 mass% or more or the like.

The prophylactic or therapeutic agent of the invention can be used in the pharmaceutical field. Moreover, the prophylactic or therapeutic agent of the invention can be used as a food additive, a health food (a functional food, a nutritional supplement (a supplement or the like), a dietary supplement, an enriched food, a balanced food or the like), a food for special dietary use, a food with health claim (a food for specified health use or a food with nutrient function claim) or the like. In the present description, a health food, a food for special dietary use, a food with health claim and the like are sometimes together referred to as a health food and the like.

In the embodiment of the pharmaceutical application, the preparation form is not particularly limited and can be various preparation forms, for example, an orally administered preparation such as tablets, pills, capsules, powder, granules and syrup, a parenterally administered preparation such as an injection (an intravenous injection, a local injection, an intramuscular injection or the like), infusion, an external agent (gel, ointment, cream, a plaster or an inhalant), a gargle and a suppository or the like. Of the preparation forms, for example, an orally administered preparation such as tablets is preferable for systemic administration, and an injection or the like is preferable for local administration.

In the embodiment, the dosage of the compound (1) or the salt thereof differs with the route of administration, the age of the patient, the body weight, the symptom or the like and cannot be generally specified, but the dosage may be such an amount that the daily dosage for an adult is generally about 5000 mg or less, preferably about 1000 mg or less. The lower limit of the dosage of the compound (1) or the salt thereof is not particularly limited, and for example, the dosage of the compound (1) can be appropriately set in the range of a daily dosage for an adult of generally 0.1 mg or more, preferably 0.5 mg or more. In the case of administration once a day, one preparation should contain the amount, and in the case of administration three times a day, one preparation should contain one third of the amount.

In the pharmaceutical application, the prophylactic or therapeutic agent of the invention is administered to a mammal or the like. Examples of the mammal include human, monkey, mouse, rat, rabbit, cat, dog, pig, cattle, horse, sheep or the like.

In the application of the health food and the like, examples of the food include dairy products such as yoghurt and milk beverage, soups, jellies, juices, coffee drinks, tea drinks, cookies, rice crackers, and cakes. The amount of the compound (1) or the salt thereof contained in the food is not particularly limited as long as it is an acceptable amount. Moreover, in the application of the health food and the like, the prophylactic or therapeutic agent of the invention may be in the form of tablets, powder, granules or the like. In the case of such a form, the amount of the compound (1) or the salt thereof contained in the tablets, the powder, the granules or the like can be appropriately set.

In the food additive application, the prophylactic or therapeutic agent of the invention can be used for adding to a food to thus add the capability of preventing or treating a disorder which can be treated by induction of proliferation or differentiation of a chondrocyte to the food. As described above, the compound (1) or the salt thereof itself may be used as the prophylactic or therapeutic agent of the invention or a composition combined with a carrier (for example, an isotonizing agent, a chelating agent, a stabilizer, a pH regulator, an antiseptic, an antioxidant, a solubilizer, a thickener or the like). The blending ratio in the use as a composition is as described above. Moreover, the added amount to the food can be set, for example, in such a manner that the amount of the compound (1) or the salt thereof contained in the food is in the range cited in the embodiment of the health food and the like.

### Agent for Inducing Proliferation or Differentiation of Chondrocyte

The compound (1) or the salt thereof, which is the active ingredient of the invention, exhibits the effect of promoting proliferation of a chondrocyte or inducing differentiation. Moreover, by inducing proliferation or differentiation of a chondrocyte, cartilage tissue can be increased. Therefore, the invention also provides an agent for inducing proliferation or differentiation of a chondrocyte containing the compound (1) or the salt thereof.

Here, the compound (1) or the salt thereof, which is the active ingredient of the invention, is known to have the effect of suppressing the function of osteoclasts that absorb the bone (NPL 1), and MPMBP, of the compound (1) or the salt thereof, is known to also have the action of promoting osteogenesis in addition to the effect. However, the effect of inducing proliferation or differentiation of a chondrocyte has not been known at all. Here, cartilage and bone are tissues which exist in close parts but are completely different in terms of the functions and the components. For example, the two are functionally different because while cartilage is a tissue having excellent resistance to mechanical pressure, a bone is a hard tissue having excellent flex resistance. Regarding the components, while the cell components of cartilage are chondroblasts and chondrocytes, the cell components of a bone are osteocytes, osteoblasts and osteoclasts. Moreover, regarding the matrix (extra cellular matrix), while the collagen fibers in cartilage are mainly type II collagen, the collagen fibers in the bone are mainly type I collagen, and the two are different also because the bone contains a large amount of inorganic calcium salts (calcium phosphate, calcium carbonate and the like).

Moreover, the two are different structurally, for example, because cartilage does not basically have developed vessels and receives nutrients through passive diffusion from the cartilage surface and because chondrocytes are dispersed in cavities (cartilage cavities) surrounded by the matrix that the chondrocytes themselves have formed and maintain the matrix while the inside of the bone is filled with the bone marrow.

Mesenchymal stem cells are known to have the ability of differentiating into osteocytes, chondrocytes, adipocytes and the like (NPL 3), and the differentiation is directed by a different specific transcription factor. As described above, MPMBP is known to also have the action of promoting osteogenesis (NPL 2). Accordingly, it is expected that differentiation from mesenchymal stem cells into osteoblasts and bone tissue is promoted by the addition of MPMBP or the like and that differentiation into cartilage is thus suppressed. The present inventors, however, have found unexpectedly that proliferation or differentiation of chondrocytes is induced. The effect of proliferating or differentiating chondrocytes of the compound (1) or the salt thereof in the invention cannot be expected from the conventional arts.

Here, the active ingredient, the preparation form, the used amount or the like of the agent for inducing proliferation or differentiation of a chondrocyte can be selected in the similar manners to those of the prophylactic or therapeutic agent of the invention. Furthermore, the agent for inducing proliferation or differentiation of a chondrocyte of the invention can also induce proliferation or differentiation of a chondrocyte *in vitro* by adding to a mesenchymal stem cell and/or a cartilage precursor cell. Accordingly, the invention also provides a method for inducing proliferation or differentiation of a chondrocyte including a step of causing the compound (1) or the salt thereof to act on a mesenchymal stem cell and/or a cartilage precursor cell *in vitro.*

The mesenchymal stem cell is a cell obtained from the cord blood, the bone marrow or the like or the like. The cartilage precursor cell is a cell obtained by induced differentiation from a mesenchymal stem cell or the like. These cells may be known cells, those produced in accordance with a known method or the like. Commercial cells or the like may be used as these cells.

In addition, these cells may be isolated cells or in the form of a cell cluster, a tissue slice or the like. Moreover, these cells may be derived from human, mouse, rat, guinea pig, monkey, dog, cat, *Xenopus laevis* or the like. The step of causing the compound (1) or the salt thereof to act can be conducted, for example, by adding to a mesenchymal stem cell and/or a cartilage precursor cell. The added amount is not particularly limited but can be set, for example, in the range of 1 to 100 µM, preferably 5 to 15 µM in the cell culture solution and is further preferably around 10 µM.

In the method of the invention, the mesenchymal stem cell and/or the cartilage precursor cell may be used in the state of being arranged in a solution such as a medium. As the medium, a medium which is generally used for culturing animal cells can be appropriately used. Examples of such a medium include DMEM medium, αMEM medium, RPMI medium, and BJG medium. Moreover, serum, a buffer, a growth factor, a differentiation factor, a stabilizer or the like may be appropriately added to the medium.

In addition, in the step, a mesenchymal stem cell and/or a cartilage precursor cell may be maintained in the presence of the compound (1) or the salt thereof. In the embodiment, during maintaining, the cell may be left still, shaken or rotated. The maintaining time is not particularly limited but can be set, for example, between 12 hours and 10 days, preferably between 36 and 72 hours. The temperature of the step is not limited, either, but can be set, for example, at 30 to 40°C, preferably around 37°C. Through the step, a chondrocyte is differentiated from the mesenchymal stem cell and/or the cartilage precursor cell and proliferated. The method of the invention may include a step of recovering the chondrocytes which are differentiated and proliferated by the above step. For the recovery step, a method which is used in the field of cell culture to which the invention belongs can be widely used. The method used for the recovery step is centrifugation, filtration, adsorption, forming in the form of a sheet, forming with a 3D printer or the like.

Specific embodiments of the invention are explained in detail below by illustrating Examples, but the invention is not limited to the Examples.

### Examples

### Example 1

Fifty 12-week-old female Sprague-Dawley rats were subjected to ovariectomy (OVX; n = 40) or to sham OVX operation (Sham; n = 10). The OVX rats were divided into four groups 12 weeks after the operation, and 0 (0.9% NaCl), 1.2, 6.0 or 30 mg/kg of MPMBP was administered once a week for 12 weeks by subcutaneous injection.

As a control, the rats of the sham group were raised under the same conditions without the administration, and the mandibular condyle was extracted at the end of the experiment.

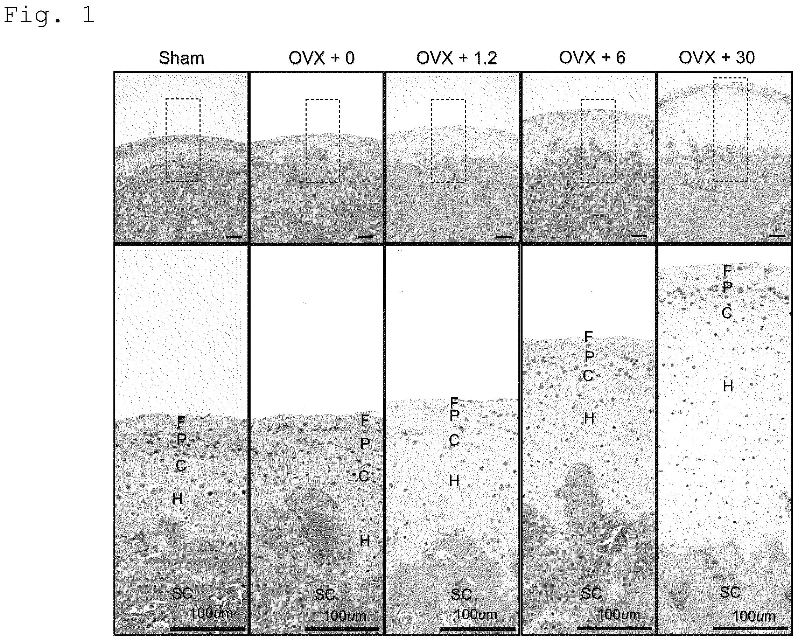

The effects of the OVX and the administration of MPMBP on the mandibular condylar cartilage are shown in Fig. 1. Using a hematoxylin-eosin-stained tissue slide of the sagittal section of the mandibular condyle, the thickest cartilage layer part in the central region was divided into four cartilage layers of the fibrous layer (F), the proliferating cell layer (P), the chondrocyte layer (C) and the hypertrophic cell layer (H) based on the method of Mizoguchi et al (2013).

Typical tissue images of the mandibular condylar cartilage which was thickened in a dose dependent manner by the administration of MPMBP are shown. A dose-dependent increase in the numbers of the hypertrophic chondrocytes and the empty lacunae is observed.

The details have been explained in the paper of Takizawa et al. (Takizawa A, Chiba M, Ota T, Yasuda M, Suzuki K, Kanemitsu T, Itoh T, Shinoda H, Igarashi K. 2016. The novel bisphosphonate disodium dihydrogen-4-[(methylthio) phenylthio] methanebisphosphonate increases bone mass in post-ovariectomy rats. J Pharmacol Sci. 131(1):37-50.), for example, in 2.2. Preparation of MPMBP for injection, 2.3 Animals and experimental schedule and the like of 2. Materials and methods in the document. The research and the OVX protocol were approved by the Animal Research Committee of Tohoku University and the Committee for Animal Experiments at Japan SLC Inc., respectively. The animal care and experiments were performed in accordance with the guidelines of the Institute for Animal Experimentation of Tohoku University Graduate School of Medicine.

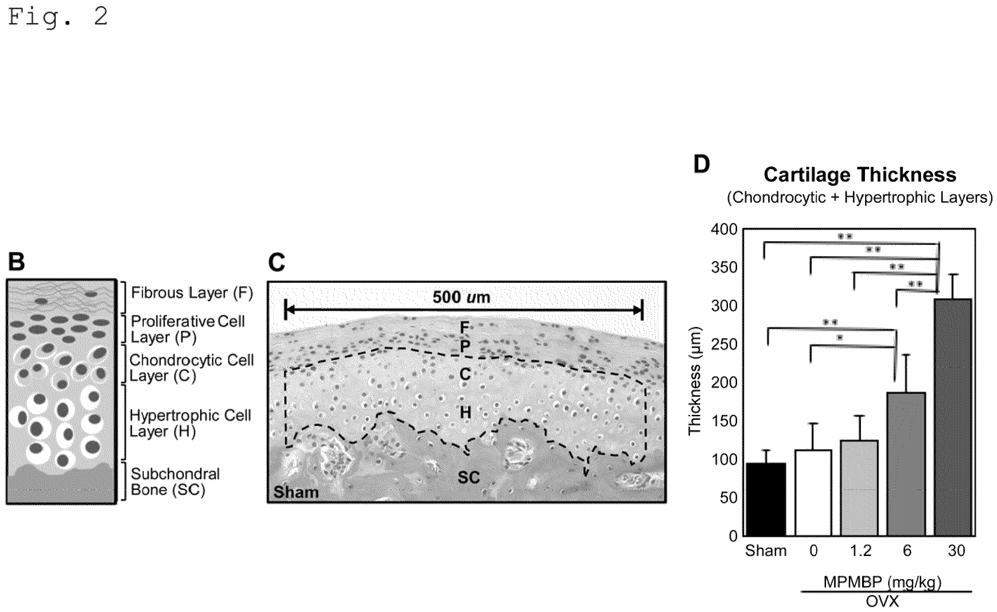

Histomorphometry was conducted regarding the effects of the OVX and the subsequent administration of MPMBP on the mandibular condylar cartilage shown in Fig. 1. The results are shown in Fig. 2.

There was no significant difference in the thickness of the mandibular condylar cartilage between the sham group and the OVX + 0 group. However, the total thickness of the chondrocyte layer and the hypertrophic cell layer increased significantly with MPMBP (OVX + 6 and OVX + 30), and thus as a result, the mandibular condylar cartilage could be thickened significantly. This suggested that MPMBP causes thickening of the cartilage layer in a dose dependent manner and can contribute to repair and protection of the mandibular condylar cartilage in an ovariectomized rat model.

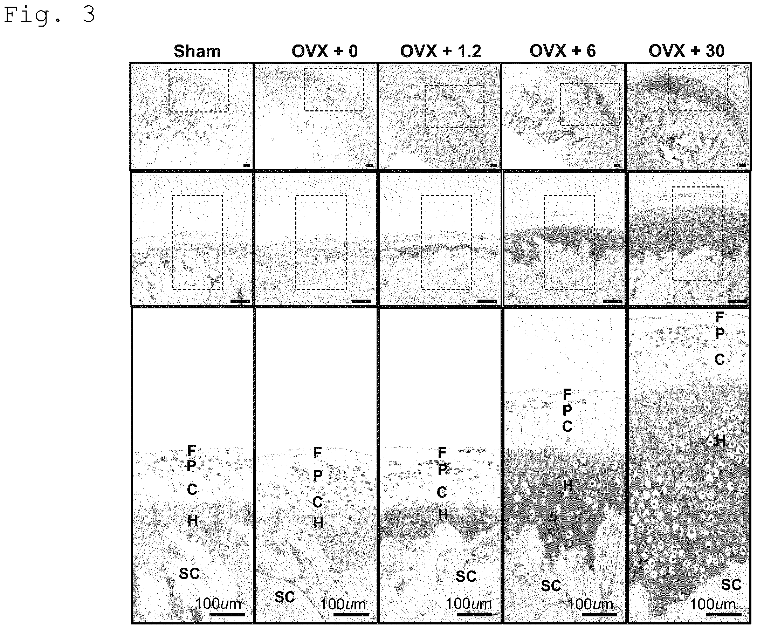

Tissue images of the toluidine blue-stained mandibular condylar cartilage are shown in Fig. 3. In Fig. 3, the magnification is x4, x10 and x20 from the top. To the OVX rats, MPMBP was administered at 0 mg/kg/week (OVX + 0), 1.2 mg/kg/week (OVX + 1.2), 6 mg/kg/week (OVX + 6) or 30 mg/kg/week (OVX + 30).

Central sagittal sectional tissue images of the thickest part of the mandibular condyle: the fibrous layer (F), the proliferating cell layer (P), the chondrocyte layer (C), the hypertrophic chondrocyte layer (H) and the subchondral bone (SC). Toluidine blue staining-positive cartilage matrices were found. It is observed that the intensity of the toluidine blue staining increased in the cartilage layer (the chondrocyte layer + the hypertrophic chondrocyte layer) which was reduced by the OVX by the administration of MPMBP and that the thickness of the stained cartilage layer increased. The scale bar is 100 µm. The above results suggest that the mandibular condylar cartilage matrix rich in proteoglycans is increased by the administration of MPMBP.

It was observed that the intensity of toluidine blue staining was low in the mandibular condylar cartilage matrix of the OVX rats, and it was observed that the expression of proteoglycans decreased (OVX + 0). Moreover, MPMBP increased the expression of proteoglycans in the mandibular condylar cartilage matrix, and staining stronger than the staining intensity observed with the sham operation rats (Sham) was seen. (OVX + 1.2, OVX + 6 and OVX + 30). In particular, the toluidine blue-positive matrix was localized in the hypertrophic chondrocyte layer of the mandibular condylar cartilage.

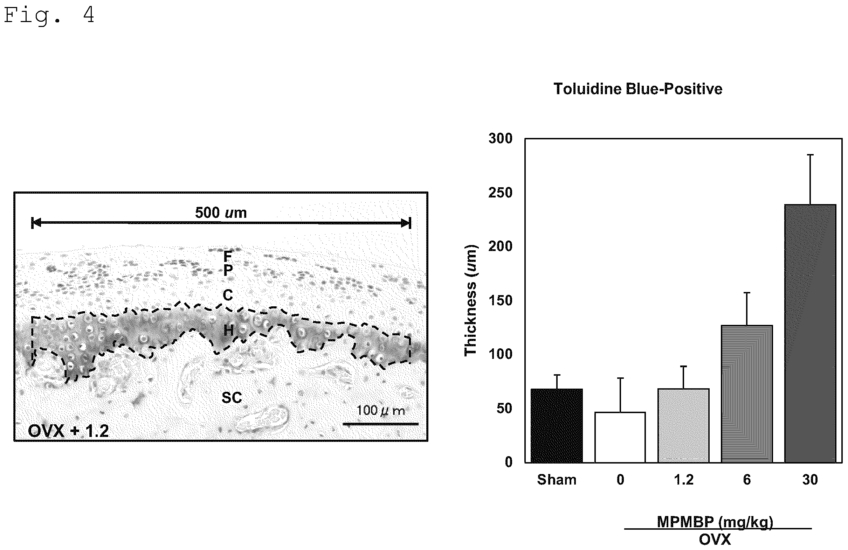

Next, the effects of the OVX and the subsequent administration of MPMBP in the mandibular condylar cartilage matrix were evaluated using toluidine blue-stained slices. The results are shown in Fig. 4. The area of the toluidine blue-positive cartilage matrix (surrounded by the dashed line) was measured with a width of 500 µm. MPMBP significantly increased the thickness of the toluidine blue-positive mandibular condylar cartilage matrix rich in proteoglycans in a dose dependent manner (OVX + 6 and OVX + 30). The findings suggested that MPMBP significantly enhances the expression of proteoglycans in the mandibular condylar cartilage matrix which has been reduced after the OVX and that the thickness of the mandibular condylar cartilage matrix rich in proteoglycans is directly proportional to a dose-dependent increase in MPMBP.

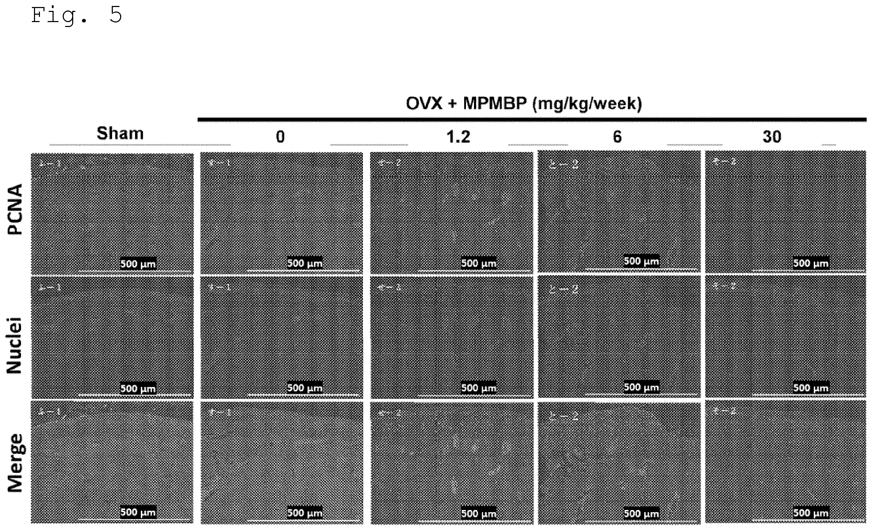

Proliferating cell nuclear antigen (PCNA) is a factor which promotes the activity of DNA polymerase ▲f▼B, and a cell can be demonstrated to be proliferating through detection of the protein by immunostaining. Immunohistochemical staining using an anti-PCNA antibody as the primary antibody was conducted, and detection with red fluorescence was conducted using a secondary antibody which was fluorescently labeled with Alexa555. All the samples were stained under the same conditions, and pictures were taken using a fluorescence microscope under the same conditions. The results are shown in Fig. 5.

Because PCNA exists in the nuclei of proliferating cells, the nuclei are stained. There are many proliferating cells in the bone marrow, and many positive cells which emit red fluorescence in PCNA immunostaining are observed in the bone marrow. PCNA-positive proliferating cells are observed in the chondrocyte layer by the administration of MPMBP. On the other hand, PCNA-positive proliferating cells are not observed in the fibrous layer, the proliferating layer and the hypertrophic cartilage layer. PCNA-positive cells, which indicate proliferation, were observed in the chondrocyte layer of the mandibular condylar chondrocytes of the rats to which MPMBP was administered.

### Example 2

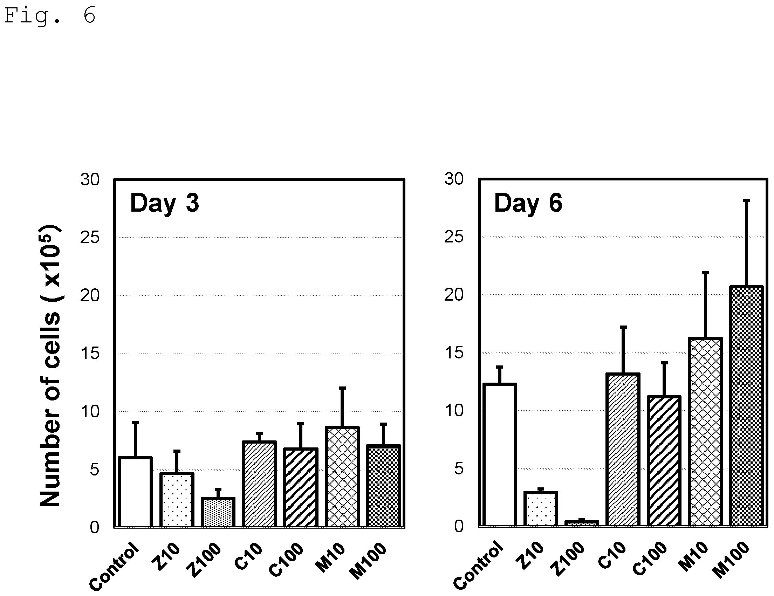

ATDC5 cells were cultured at a cell density of 5×10⁵ cells/35 mm dish. As the culture solution, DMEM-Ham's F12 medium containing 5% FBS and 1% Pen/Strep was used. Adjustment was conducted to final concentrations of MPMBP of 10 µM (M10) and 100 µM (M100), clodronate of 10 µM (C10) and 100 µM (C100), zoledronate of 10 µM (Z10) and 100 µM (Z100) and a control (Vehicle), and the cells were cultured under the conditions of 5% CO₂ at 37°C. The medium was changed every three days. The results are shown in Fig. 6.

On the third day of culture, the numbers of the BP-treated cells were all smaller than that of the control. The number decreased in Z10 and Z100 but increased with C10, C100, M10 and M100 treatment.

On the sixth day of culture, the number of cells decreased significantly in Z10 and Z100 compared to those of the control, C10, C100, M10 and M100 but increased in particular with M10 and M100 treatment.

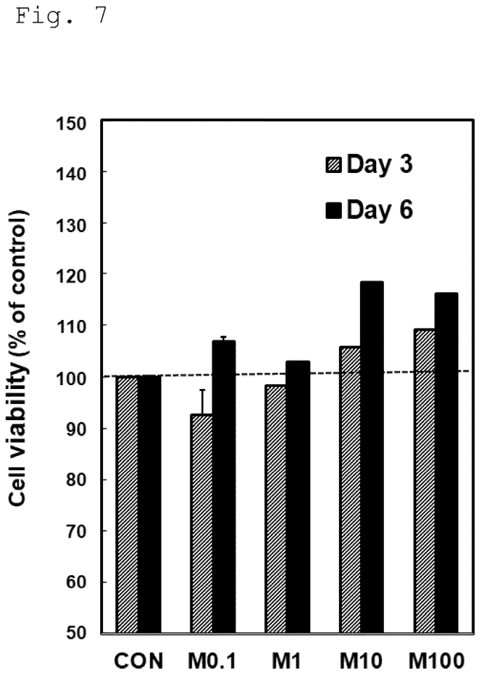

Next, ATDC5 cells were cultured at a cell density of 5×10⁵ cells/35 mm dish. As the culture solution, DMEM-Ham's F12 medium containing 5% FBS and 1% Pen/Strep was used. Adjustment was conducted to final concentrations of MPMBP of 0.1 µM (M0.1), 1 µM (M1), 10 µM (M10) and 100 µM (M100) and a control (Vehicle), and the cells were cultured under the conditions of 5% CO₂ at 37°C. The medium was changed every three days. Alamar Blue assay was conducted after three days of culture or six days of culture. After incubation under the conditions of 37°C and 5% CO₂ for three hours, measurement was made with an excitation wavelength of 544 nm and a measurement wavelength of 590 nm. The results are shown in Fig. 7.

On the third day of culture, the viability of the cells increased with M10 and M100 treatment.

On the sixth day of culture, the viability of the cells increased with M0.1, M1, M10 and M100 treatment.

### Example 3

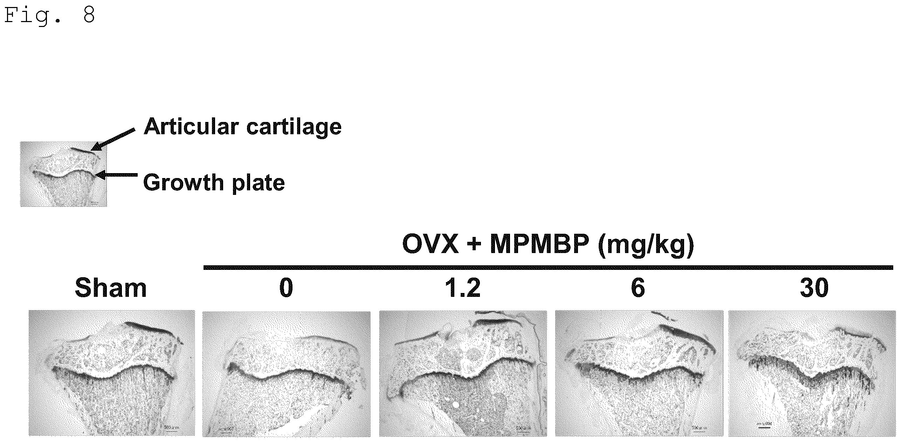

Twelve-week-old female SD rats were subjected to ovariectomy (OVX) or to sham operation (Sham), raised with administration of MPMBP once a week (1.2, 6 or 30 mg/kg/week or 0.9% NaCl (OVX) as a control) for 12 months from the age of 24 weeks to 36 weeks and sacrificed at the age of 36 weeks, and the extracted tibiae were stained with toluidine blue according to a report. The results are shown in Fig. 8.

An increase in the toluidine blue-positive cartilage layer which is as significant as that in the mandibular condylar cartilage is not found in the tibia. However, an increase in the cartilage layer is found both in the articular cartilage and the growth plate cartilage of the tibia.

The difference is believed to be caused because the mandibular condylar cartilage is a non-load bearing bone to which no pressure is applied at rest and thus the mechanical environment is different from that of the tibia to which the body weight is applied. Moreover, in the developmental process, the mandibular condylar cartilage is cartilage generated in the joint part of the mandible formed through the process of a membrane bone while the tibia is a bone formed through the process of a cartilaginous bone, and thus, reactivity may be different. It was suggested that both cartilage tissues increase.
PCNA: Regarding proliferating cell nuclear antigen (PCNA), immunohistochemical staining using an anti-PCNA antibody as the primary antibody was conducted, and staining with red fluorescence was conducted using a secondary antibody which was fluorescently labeled with Alexa555.
Nuclei: The nuclei were stained with blue fluorescence using DAPI.
Merge: The PCNA staining and the DAPI staining were merged.

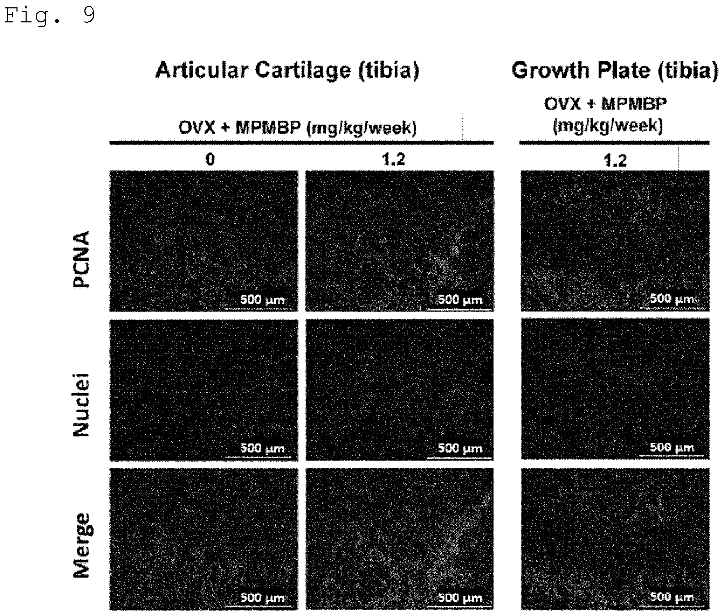

All the samples were stained under the same conditions, and pictures were taken using a fluorescence microscope under the same conditions. The results are shown in Fig. 9.

Because PCNA exists in the nuclei of proliferating cells, the nuclei are stained. There are many proliferating cells in the bone marrow, and many positive cells which emit red fluorescence with PCNA immunostaining are observed in the bone marrow. PCNA-positive proliferating cells are observed in the chondrocyte layer in the surface layer of the articular cartilage and the growth plate cartilage of the tibia by the administration of MPMBP (1.2 mg/kg/week). On the other hand, PCNA-positive proliferating cells are not observed in the cartilage layer of the OVX rats. It was suggested that chondrocytes of the articular cartilage and the growth plate cartilage of the tibia are proliferated by the administration of MPMBP.

### Industrial Applicability

According to the invention, proliferation of a chondrocyte can be promoted, or differentiation can be induced using the compound (1) or a salt thereof. Accordingly, the invention is useful for prevention or treatment of a disorder which can be treated by induction of proliferation or differentiation of a chondrocyte. Moreover, the invention is also useful for test research of chondrocytes and the like.

## Claims

1. An agent for inducing proliferation or differentiation of a chondrocyte comprising a compound represented by the general formula (1) or a salt thereof 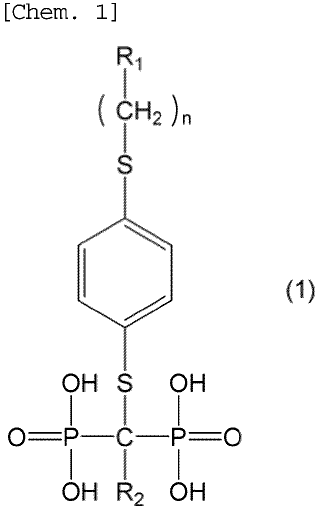 [wherein in the formula, R₁ represents a hydrocarbon group of C1 to C5 which may be substituted, R₂ represents hydrogen or a hydroxy group, and n represents an integer of 0 to 3].

2. A prophylactic or therapeutic agent for a disorder which can be treated by induction of proliferation or differentiation of a chondrocyte comprising a compound represented by the general formula (1) or a salt thereof 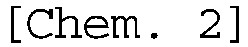 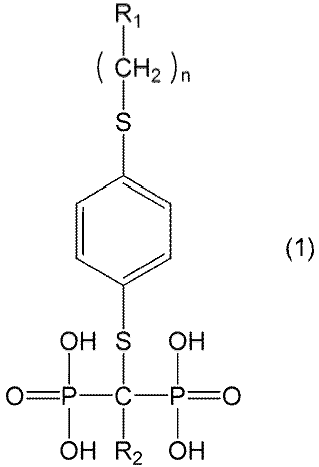 [wherein in the formula, R₁ represents a hydrocarbon group of C1 to C5 which may be substituted, R₂ represents hydrogen or a hydroxy group, and n represents an integer of 0 to 3].

3. The prophylactic or therapeutic agent according to claim 2, wherein the disorder which can be treated by induction of proliferation or differentiation of a chondrocyte is osteoarthritis, inflammatory arthritis, cartilage damage, congenital cartilage defect, an age-related decrease in articular cartilage or chondrodystrophy.

4. The prophylactic or therapeutic agent according to claim 2 or 3 which is systemically administered or locally administered.

5. The agent for inducing proliferation or differentiation of a chondrocyte according to claim 1 or the prophylactic or therapeutic agent according to claim 2 or 3, wherein the compound represented by the general formula (1) or the salt thereof is [4-(methylthio)phenylthio]-methanebisphosphonic acid or a salt thereof.

6. A method for inducing proliferation or differentiation of a chondrocyte comprising a step of causing a compound represented by the general formula (1) or a salt thereof to act on a mesenchymal stem cell and/or a cartilage precursor cell *in vitro* 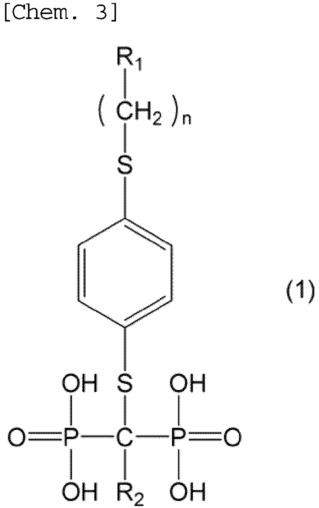 [wherein in the formula, R₁ represents a hydrocarbon group of C1 to C5 which may be substituted, R₂ represents hydrogen or a hydroxy group, and n represents an integer of 0 to 3].

7. Use of a compound represented by the general formula (1) or a salt thereof for the manufacture of a prophylactic or therapeutic agent for a disorder which can be treated by induction of proliferation or differentiation of a chondrocyte: 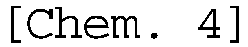 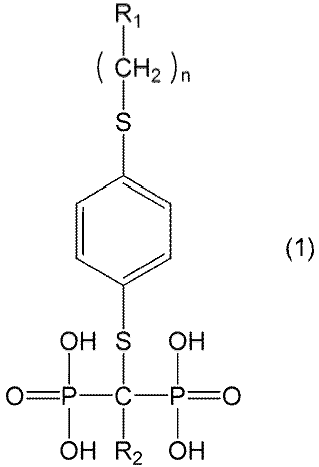 [wherein in the formula, R₁ represents a hydrocarbon group of C1 to C5 which may be substituted, R₂ represents hydrogen or a hydroxy group, and n represents an integer of 0 to 3].

8. A compound represented by the general formula (1) or a salt thereof for use in the prevention or the treatment of a disorder which can be treated by induction of proliferation or differentiation of a chondrocyte: 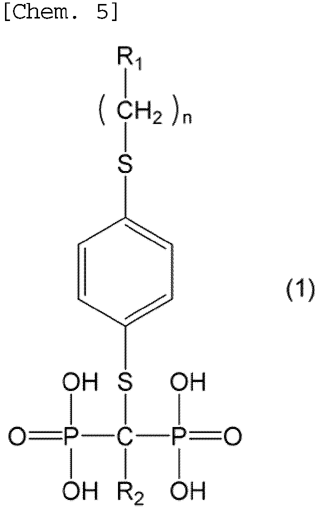 [wherein in the formula, R₁ represents a hydrocarbon group of C1 to C5 which may be substituted, R₂ represents hydrogen or a hydroxy group, and n represents an integer of 0 to 3].
